# EUROPEAN PATENT APPLICATION

(11) **EP 4 397 766 A1**
(43) Date of publication of application: **10.07.2024**
(21) Application number: 22864632.9
(22) Date of filing: 31.08.2022
(51) Int. Cl.: C12N 15/86, A61K 35/76, A61P 21/02, C12N 5/10, C12N 7/01, C07K 16/40, C07K 16/46, C12N 15/13

(54) **PHARMACEUTICAL COMPOSITION UTILIZING BORNAVIRUS VECTOR**

(30) Priority: 02.09.2021 JP 2021143184
(71) Applicant: Kyoto University, Kyoto-shi, Kyoto 606-8501 (JP)
(72) Inventor: TOMONAGA, Keizo, Kyoto-shi, Kyoto 606-8501 (JP); MAKINO, Akiko, Kyoto-shi, Kyoto 606-8501 (JP); SAKAI, Madoka, Kyoto-shi, Kyoto 606-8501 (JP); KOMORIZONO, Ryo, Kyoto-shi, Kyoto 606-8501 (JP); URUSHITANI, Makoto, Otsu-shi, Shiga 520-2192 (JP); MORIMURA, Toshifumi, Otsu-shi, Shiga 520-2192 (JP); MINAMIYAMA, Sumio, Otsu-shi, Shiga 520-2192 (JP)
(74) Representative: Studio Torta S.p.A.
(86) International application number: PCT/JP2022/032784
(87) International publication number: WO 2023/033050

(57) **Abstract**

Disclosed are a bornavirus vector comprising a nucleic acid encoding an antibody or antibody fragment capable of binding to a mutant SOD1 protein, the antibody or antibody fragment comprising a heavy-chain variable region comprising a heavy-chain CDR1 consisting of an amino acid sequence GFSLNTSGMG (SEQ ID NO: 1), a heavy-chain CDR2 consisting of an amino acid sequence IWWDDDK (SEQ ID NO: 2), and a heavy-chain CDR3 consisting of an amino acid sequence ARLGYAMDY (SEQ ID NO: 3), the heavy-chain variable region optionally having 3 or fewer amino acid substitutions, and/or a light-chain variable region comprising a light-chain CDR1 consisting of an amino acid sequence QNVGGTN (SEQ ID NO: 4), a light-chain CDR2 consisting of an amino acid sequence SAS, and a light-chain CDR3 consisting of an amino acid sequence QQYYIYPYT (SEQ ID NO: 5), the light-chain variable region optionally having 3 or fewer amino acid substitutions; a recombinant virus comprising RNA encoded by the bornavirus vector; a cell infected with the recombinant virus; and a pharmaceutical composition comprising the cell.

## Description

### Technical Field

The present invention relates to a bornavirus vector, a recombinant virus comprising RNA encoded by the bornavirus vector, and a cell infected with the recombinant virus, and a pharmaceutical composition comprising the cell.

### Background Art

Amyotrophic lateral sclerosis (ALS) is a neurodegenerative disease with onset at 60 to 70 years of age whose main symptoms are general muscular atrophy and muscle weakness, leading to fatal dysphagia and respiratory failure within 4 years after onset. 200 of ALS patients have familial ALS, and 30% of them have mutations in the Cu/Zn superoxide dismutase I (SOD1) gene. There are as many as 200 kinds of mutations. It is known that mutant SOD1 proteins abnormally accumulate in ALS.

SOD1 is an enzyme that removes an active oxygen species called "superoxide." However, the neurotoxicity caused by mutant SOD1 is unrelated to anti-active oxygen activity, and removal of mutant proteins is a major strategy in drug discovery and development. In recent years, antisense oligonucleotide therapy for SOD1 has been put into practical use, and we are entering an era in which intractable ALS can benefit from advanced medical treatment aimed at arresting onset and stopping progression. Accordingly, early diagnosis is becoming increasingly important.

Various antibodies have been reported for use in diagnosis and treatment of ALS (e.g., Patent Literature (PTL) 1 to 4 and Non-patent Literature (NPL) 1).

Borna disease virus (also referred to below as "BDV") is a neurotropic virus belonging to the order Mononegavirales that has non-segmented, negative-sense, single-stranded RNA as a genome. Furthermore, BDV replicates in cell nuclei and has characteristics that its infection is non-cytotoxic and persistent for a long period and that a wide range of hosts can be infected.

### Citation List

### Patent Literature

PTL 1: WO2011/142461
PTL 2: WO2012/058220
PTL 3: WO2014/191493
PTL 4: WO2007/025385

### Non-patent Literature

NPL 1: PLOS ONE 8(4): e61210. Doi:10.1371/journal.pone.0061210

### Summary of Invention

### Technical Problem

An object of the present invention is to provide a bornavirus vector that enables the treatment and prevention of ALS with mutations in the SOD1 gene.

Another object of the present invention is to provide a recombinant virus comprising RNA encoded by the bornavirus vector, a cell infected with the recombinant virus, and a pharmaceutical composition comprising the cell.

### Solution to Problem

The present inventors conducted extensive research to achieve the above objects, and found that regarding the antibody capable of binding to a mutant SOD1 protein obtained by the inventors, when the scFv gene is introduced into oligodendrocyte progenitor cells (OPCs) by using a bornavirus vector, and the cells are transplanted into model rats, the effects of suppressing the progression of ALS and extending life are observed. The inventors also confirmed that by introducing the scFv gene into human mesenchymal stem cells by using a bornavirus vector, the scFv can be secreted in the culture supernatant and that the integration of scFv DNA into the genome is extremely low.

The present invention has been accomplished as a result of further research based on these findings and provides the following bornavirus vector, a recombinant virus comprising RNA encoded by the bornavirus vector, a cell infected with the recombinant virus, and a pharmaceutical composition comprising the cell.

### Item 1.

A bornavirus vector comprising a nucleic acid encoding an antibody or antibody fragment capable of binding to a mutant SOD1 protein,
the antibody or antibody fragment comprising:
a heavy-chain variable region comprising a heavy-chain CDR1 consisting of an amino acid sequence GFSLNTSGMG (SEQ ID NO: 1), a heavy-chain CDR2 consisting of an amino acid sequence IWWDDDK (SEQ ID NO: 2), and a heavy-chain CDR3 consisting of an amino acid sequence ARLGYAMDY (SEQ ID NO: 3), the heavy-chain variable region optionally having 3 or fewer amino acid substitutions; and/or
a light-chain variable region comprising a light-chain CDR1 consisting of an amino acid sequence QNVGGTN (SEQ ID NO: 4), a light-chain CDR2 consisting of an amino acid sequence SAS, and a light-chain CDR3 consisting of an amino acid sequence QQYYIYPYT (SEQ ID NO: 5), the light-chain variable region optionally having 3 or fewer amino acid substitutions.

### Item 2.

The bornavirus vector according to Item 1, wherein the antibody fragment is a Fab, Fab', F(ab')₂, Fv, scFab, scFv, dsFv, ds-scFv, diabody, triabody, tetrabody, or minibody.

### Item 3.

The bornavirus vector according to Item 1 or 2, wherein the antibody fragment is an scFv.

### Item 4.

The bornavirus vector according to any one of Items 1 to 3, wherein the antibody or antibody fragment is a humanized antibody or antibody fragment.

### Item 5.

The bornavirus vector according to any one of claims 1 to 4, comprising:
(a) cDNA of recombinant viral RNA having at least the N gene, X gene, P gene, and L gene of a Borna disease virus genome in the same order as in the Borna disease virus genome and having a sequence in which the nucleic acid is inserted into the untranslated region ligated downstream of the coding region of the P gene;
(b) DNA encoding a ribozyme; and
(c) a promoter sequence,
wherein (b) is located upstream and downstream of (a), and (a) and (b) are located downstream of (c).

### Item 6.

The bornavirus vector according to Item 5, wherein (a) the cDNA of recombinant viral RNA has a sequence in which the G gene is disrupted in the Borna disease virus genome and has a sequence in which the nucleic acid is inserted into the untranslated region ligated downstream of the coding region of the P gene.

### Item 7.

The bornavirus vector according to Item 5, wherein (a) the cDNA of recombinant viral RNA has a sequence in which the G gene and M gene are disrupted in the Borna disease virus genome and has a sequence in which the nucleic acid is inserted into the untranslated region ligated downstream of the coding region of the P gene.

### Item 8.

The bornavirus vector according to Item 5, wherein (a) the cDNA of recombinant viral RNA is cDNA encoding a recombinant Borna disease virus genome in which the nucleic acid is inserted into the untranslated region between the coding region of the P gene and the coding region of the M gene.

### Item 9.

The bornavirus vector according to any one of Items 5 to 8, wherein (c) the promoter sequence is an RNA polymerase II promoter sequence.

### Item 10.

The bornavirus vector according to any one of Items 5 to 9, wherein cDNA encoding (b1) hammerhead ribozyme is located upstream of (a) the cDNA of recombinant viral RNA, and a cDNA sequence encoding (b2) hepatitis delta virus ribozyme is located downstream of (a).

### Item 11.

A recombinant virus comprising RNA encoded by the bornavirus vector of any one of Items 1 to 10.

### Item 12.

A cell into which the nucleic acid has been introduced by infection with the recombinant virus of Item 11.

### Item 13.

The cell according to Item 12, wherein the cell is an oligodendrocyte progenitor cell, glial neural progenitor cell, dopamine neural progenitor cell, microglia, neural progenitor cell, neural stem cell, mesenchymal stem cell, Muse cell, iPS cell, or ES cell.

### Item 14.

A pharmaceutical composition comprising the cell of Item 12 or 13.

### Item 15.

The pharmaceutical composition according to Item 14, which is for use in the treatment and/or prevention of amyotrophic lateral sclerosis.

### Item 16.

A method for treating a disease in which mutant SOD1 accumulates, such as amyotrophic lateral sclerosis, comprising administering an effective amount of the cell of Item 12 or 13 to a mammal.

### Item 17.

Use of the cell of Item 12 or 13 for the production of a pharmaceutical composition for treating and/or preventing a disease in which mutant SOD1 accumulates, such as amyotrophic lateral sclerosis.

### Advantageous Effects of Invention

By using the bornavirus vector of the present invention, a recombinant virus comprising RNA encoded by the bornavirus vector can be produced, and further, by infecting cells with the recombinant virus, cells expressing an antibody or antibody fragment capable of binding to a mutant SOD1 protein can be created. The transplantation of the cells enables the treatment and prevention of ALS with mutations in the SOD1 gene.

### Brief Description of Drawings

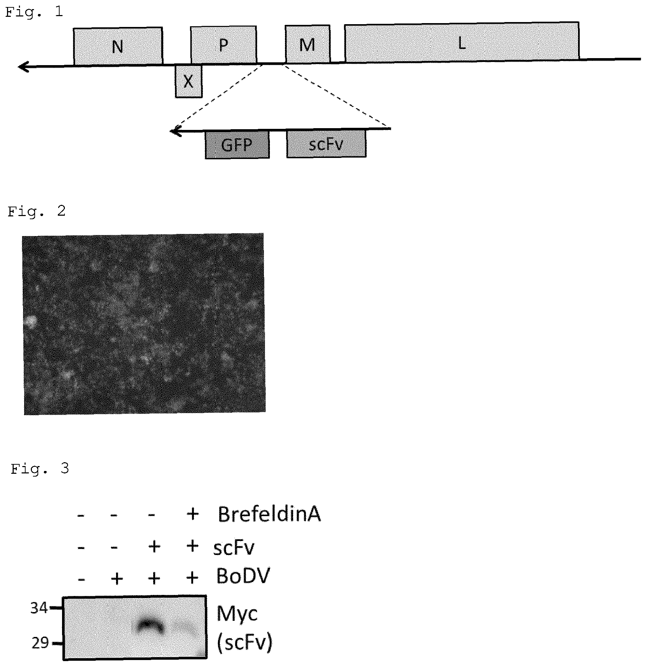

Fig. 1 shows the construction of the pCAG-Fct p/mΔG LL GFP-scFv plasmid produced in the Examples, into which an anti-SOD1 antibody gene is inserted.
Fig. 2 is a photograph showing the results of observing OPCs inoculated with a bornavirus vector encoding an scFv by using a fluorescence microscope.
Fig. 3 is a photograph showing the results of western blotting of culture supernatants of OPCs into which each bornavirus vector encoding an scFv was introduced.

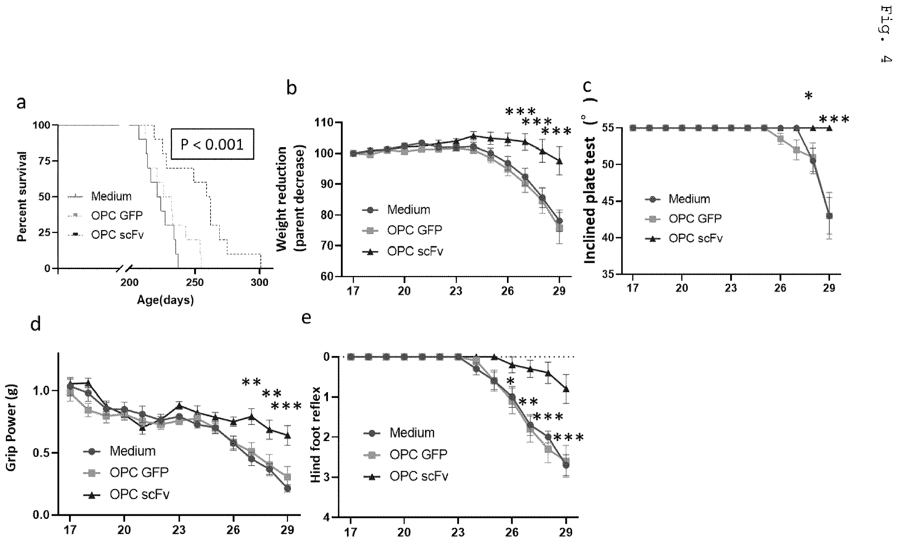

Fig. 4 shows graphs indicating the results of (A) a survival rate test, (B) measurement of body weight change, (C) an inclined plate test, (D) a grip strength test, and (E) a hindlimb reflex test on mutant SOD1 transgenic mice to which OPCs into which each bornavirus vector was introduced was administered. Data are presented as mean ± SD, * P<0.01, ** P<0.001, *** P<0.0001, two-way ANOVA.

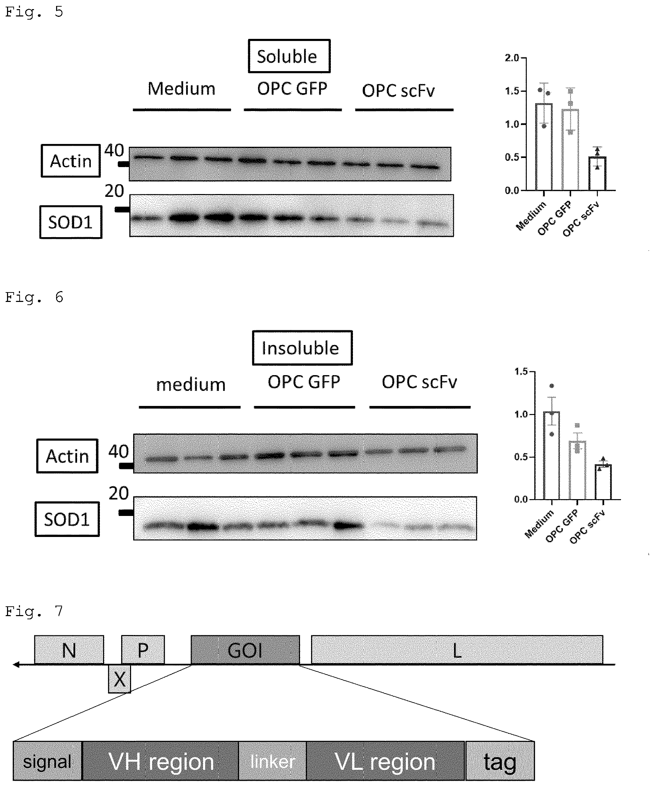

Fig. 5 show the results of relative quantitation of the amounts of SOD1 in the lumbar spinal cord collected from mutant SOD1 transgenic mice into which OPCs were transplanted, by western blotting (soluble fraction). Left: western blotting; right: graph showing the amount of SOD1 in each OPC administration group on the assumption that SOD1/beta-actin in the NI group is 1.0. Data are presented as mean ± SD, n=3.
Fig. 6 show the results of relative quantitation of the amounts of SOD1 in the lumbar spinal cord collected from mutant SOD1 transgenic mice into which OPCs were transplanted, by western blotting (insoluble fraction). Left: western blotting; right: graph showing the amount of SOD1 in each OPC administration group on the assumption that SOD1/beta-actin in the NI group is 1.0. Data are presented as mean ± SD, n=3.
Fig. 7 shows the construction of the pCAG-FctHe80 p/lΔMG scFv plasmid produced in the Examples, into which an anti-SOD1 antibody gene is inserted.

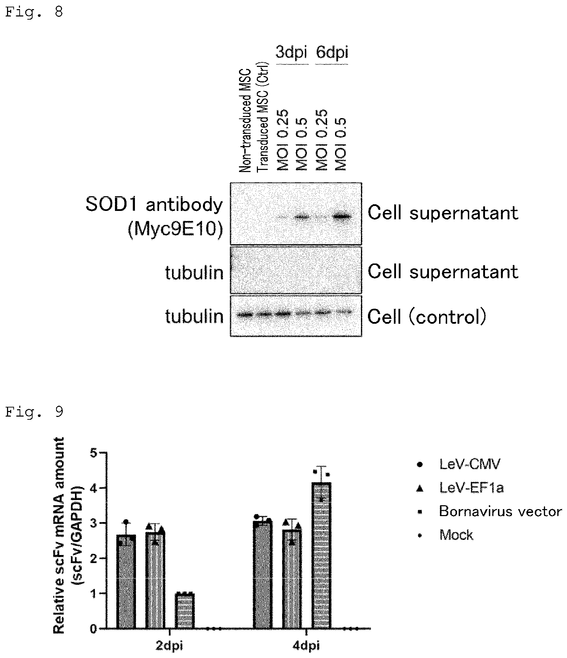

Fig. 8 shows photographs indicating the results of western blotting of culture supernatants of mesenchymal stem cells into which each bornavirus vector encoding an scFv was introduced.
Fig. 9 is a graph showing the relative amount of scFv mRNA to GAPDH, expressed after the introduction of each virus vector encoding an scFv into mesenchymal stem cells. The virus vectors used were as follows: LeV-CMV: CSII-CMV scFv, LeV-EF1a: CSII-EF scFv, and bornavirus vector: pCAG-FctHe80 p/lΔMG scFv. Data are presented as mean ± SD, n=3.

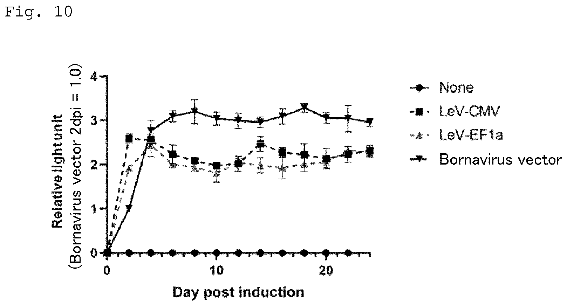

Fig. 10 is a graph showing the relative luminescence of an scFv secreted after the introduction of each virus vector encoding the scFv into mesenchymal stem cells. The virus vectors used were as follows: LeV-CMV: CSII-CMV scFv, LeV-EF1a: CSII-EF scFv, and bornavirus vector: pCAG-FctHe80 p/lΔMG scFv. Data are presented as mean ± SD, n=3.

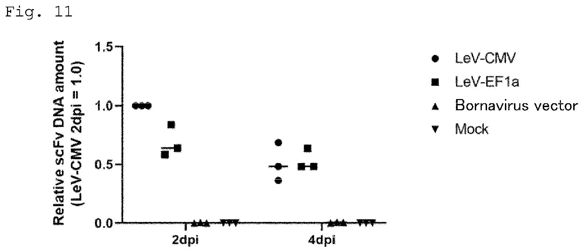

Fig. 11 is a graph showing the relative amount of scFv DNA detected after the introduction of each virus vector encoding an scFv into mesenchymal stem cells. The virus vectors used were as follows: LeV-CMV: CSII-CMV scFv, LeV-EF1a: CSII-EF scFv, and bornavirus vector: pCAG-FctHe80 p/lΔMG scFv. Data are presented as mean, n=3.

### Description of Embodiments

Embodiments of the present invention are described in detail below.

In the present specification, the term "comprise" also encompasses the meanings of "essentially consisting of" and "consisting of."

In the present invention, the term "nucleic acid" means RNA or DNA. In the present invention, the term "gene" includes double-stranded DNA, single-stranded DNA (sense strand or antisense strand), and fragments thereof, unless otherwise specified. Further, in the present invention, the term "gene" refers to a regulatory region, a coding region, an exon, and an intron, without distinction, unless otherwise specified.

In the present invention, the terms "nucleic acid," "nucleotide," and "polynucleotide" have the same meaning and include both DNA and RNA, which may be double-stranded or single-stranded.

In the present specification, the term "SOD1" means protein unless otherwise stated. However, when it is appropriate that the term "SOD1" be interpreted as gene, the term "SOD1" means gene.

The RefSeq IDs below are registered on the NCBI website.

The bornavirus vector of the present invention is characterized by comprising a nucleic acid (also referred to below as "the nucleic acid of the present invention") encoding an antibody or antibody fragment (also referred to below as "the antibody or antibody fragment of the present invention") capable of binding to a mutant SOD1 protein,
the antibody or antibody fragment comprising:
a heavy-chain variable region comprising a heavy-chain CDR1 consisting of an amino acid sequence GFSLNTSGMG (SEQ ID NO: 1), a heavy-chain CDR2 consisting of an amino acid sequence IWWDDDK (SEQ ID NO: 2), and a heavy-chain CDR3 consisting of an amino acid sequence ARLGYAMDY (SEQ ID NO: 3), the heavy-chain variable region optionally having 3 or fewer amino acid substitutions; and/or
a light-chain variable region comprising a light-chain CDR1 consisting of an amino acid sequence QNVGGTN (SEQ ID NO: 4), a light-chain CDR2 consisting of an amino acid sequence SAS, and a light-chain CDR3 consisting of an amino acid sequence QQYYIYPYT (SEQ ID NO: 5), the light-chain variable region optionally having 3 or fewer amino acid substitutions.

Superoxide dismutase (SOD) is an enzyme that catalyzes the dismutation of superoxide anion to oxygen and hydrogen peroxide (EC 1.15.1.1). In humans, three forms of SOD are present. SOD1 is present in the cytoplasm and is generally a dimer. SOD2 is present in the mitochondria, SOD3 is present extracellularly, and these are generally tetramers. SOD1 and SOD3 contain copper and zinc in the reactive center, and SOD2 contains manganese in the reactive center.

SOD1 in the present invention is generally derived from an animal, preferably derived from a mammal, and particularly preferably derived from a human.

The amino acid sequence of human-derived SOD1 protein is registered as RefSeq Accession No. NP_000445 and is represented by SEQ ID NO: 6. Moreover, the gene encoding human-derived SOD1 protein is registered as RefSeq Accession No. NM_000454, and its base sequence is represented by SEQ ID NO: 7.

The mutant SOD1 in the present invention refers to SOD1 folded into a structure different from the natural structure due to mutation in the protein by, for example, amino acid substitution, deletion, insertion, or addition, and particularly refers to SOD1 having a pathogenic structure associated with ALS. Mutant SOD1 may be due to mutation in the protein sequence or may be due to other modifications. In addition, the mutant SOD1 may have sporadic mutation or familial mutation. In particular, the mutant SOD1 is SOD1 having familial mutation.

The complementarity determining region (CDR) contained in the antibody or antibody fragment of the present invention is characterized by being capable of binding to mutant SOD1. It is desirable that the antibody or antibody fragment of the present invention specifically binds to mutant SOD1 and does not bind to wild-type SOD1 having the normal structure.

The antibody or antibody fragment of the present invention preferably comprises:
a heavy-chain variable region comprising a heavy-chain CDR1 consisting of an amino acid sequence GFSLNTSGMG (SEQ ID NO: 1), a heavy-chain CDR2 consisting of an amino acid sequence IWWDDDK (SEQ ID NO: 2), and a heavy-chain CDR3 consisting of an amino acid sequence ARLGYAMDY (SEQ ID NO: 3), the heavy-chain variable region optionally having 3 or fewer amino acid substitutions; and
a light-chain variable region comprising a light-chain CDR1 consisting of an amino acid sequence QNVGGTN (SEQ ID NO: 4), a light-chain CDR2 consisting of an amino acid sequence SAS, and a light-chain CDR3 consisting of an amino acid sequence QQYYIYPYT (SEQ ID NO: 5), the light-chain variable region optionally having 3 or fewer amino acid substitutions.

Amino acid substitution is performed so that the binding of the antibody or antibody fragment to mutant SOD1 is maintained. The phrase "optionally having 3 or fewer amino acid substitutions" means that a total of 3 or fewer amino acids may be substituted in the heavy-chain variable region or light-chain variable region. Amino acid substitution is preferably performed in the CDR.

The number of amino acid substitutions is preferably 2 or fewer, more preferably 1 or fewer, and even more preferably 0. When amino acid substitution is performed, the activity of the original antibody fragment is thought likely to be maintained by substitution with an amino acid having similar properties. Techniques for amino acid substitution in a specific amino acid sequence are known.

The antibody fragment refers to a portion of an antibody that retains some or all of the binding activity of the antibody from which the antibody fragment is derived. Examples of antibody fragments include a Fab, Fab', F(ab')₂, Fv, scFab, scFv, dsFv, ds-scFv, diabody, triabody, tetrabody, minibody, and the like. Diabodies, triabodies, tetrabodies, and minibodies may be of the same type or a combination of different types.

The antibody fragment of the present invention is preferably an scFv. "scFv" refers to an antibody fragment in which Fv comprising a heavy-chain variable region and a light-chain variable region is connected by an appropriate peptide linker. The scFv can be one in which VH and VL are connected in any order.

The sequence of the framework region (FR region) in the variable region of the antibody or antibody fragment of the present invention is not particularly limited as long as the antibody fragment is capable of binding to mutant SOD1, and any sequence can be used. The antibody or antibody fragment of the present invention is preferably a humanized antibody or antibody fragment having a human-derived framework region. Such a humanized antibody or antibody fragment can be prepared by a known method.

The antibody or antibody fragment of the present invention may be derived from any animal, such as mice, rats, guinea pigs, rabbits, bovine, goats, and sheep, and is not particularly limited. The isotype of the antibody or antibody fragment of the present invention is not particularly limited, and may be IgG (IgG1, IgG2, IgG3, and IgG4), IgA, IgD, IgE, or IgM. Further, any functional peptide domain (e.g., a signal sequence or a blood-brain barrier permeable peptide) or the like may be added to the N-terminus and/or C-terminus of the antibody or antibody fragment of the present invention.

The antibody or antibody fragment of the present invention can recognize a wide variety of mutant SOD1 proteins regardless of the site of amino acid substitution, and has a wide range of applications as an antibody or antibody fragment for the prevention and treatment of ALS with mutations in the SOD1 gene. For example, the antibody or antibody fragment of the present invention can recognize mutant SOD1 proteins, such as A4V, C6G, H46R, G85R, G93A, C111Y, I112T, and I113T. Antisense oligonucleotides require mutant gene-specific treatment, but the antibody or antibody fragment of the present invention can be used for the prevention and treatment regardless of the type of mutation.

The nucleic acid of the present invention is characterized by encoding the antibody or antibody fragment described above. In the nucleic acid, a sequence or the like for improving translation and RNA stability may be added to untranslated regions of mRNA to be transcribed. The nucleic acid can be prepared by a conventional method, such as PCR, chemical synthesis, or biochemical cleavage/recombination.

The bornavirus vector of the present invention is for producing the antibody or antibody fragment described above and is characterized by comprising the nucleic acid described above. The bornavirus vector of the present invention may also contain a gene other than the nucleic acid described above. The bornavirus vector is not particularly limited, and a wide range of known bornavirus vectors can be used (see, for example, WO2010/113647; JP2018-148865A; Microbiol Immunol 2017 Sep; 61(9):380-386; J Virol. 2011 Dec; 85(23):12170-8; and Sci Rep. 2016 May 18; 6:26154). The above nucleic acid can be inserted into the vector by a known method. The bornavirus vector can maintain semi-permanent infection because the virus is present as extrachromosomal RNA (episomal) in the cell nucleus and localized to chromosomes even in dividing and proliferating cells. The N, P, X, and L proteins (derived from the virus) and a gene of interest (RNA of interest) are constitutively expressed in cells.

The BDV genome encodes at least six proteins: nucleoprotein (N protein), X protein, phosphoprotein (P protein), matrix protein (M protein), surface glycoprotein (G protein), and RNA-dependent RNA polymerase (L protein). The BDV genome has the N, X, P, M, G, and L genes in this order from the 3' end. In the present specification, viral RNAs encoding the G protein, M protein, N protein, P protein, and L protein are referred to as the "G gene," "M gene," "N gene," "P gene," and "L gene," respectively.

For example, bornavirus vectors and their preparation method, recombinant viruses containing RNA encoded by a bornavirus vector and their preparation method, and the method of infecting cells with the virus are well known. For example, reference may be made to WO2010/113647; JP2018-148865A; Microbiol Immunol 2017 Sep; 61(9):380-386; J Virol. 2011 Dec; 85(23):12170-8; Sci Rep. 2016 May 18; 6:26154, and the like.

Specific examples of the bornavirus vector of the present invention include a bornavirus vector comprising (a) cDNA of recombinant viral RNA having at least the N gene, X gene, P gene, and L gene of the Borna disease virus genome in the same order as in the Borna disease virus genome and having a sequence in which the nucleic acid of the present invention is inserted into the untranslated region ligated downstream of the coding region of the P gene, (b) DNA encoding a ribozyme, and (c) a promoter sequence, wherein (b) is located upstream and downstream of (a), and (a) and (b) are located downstream of (c).

(a) The cDNA of recombinant viral RNA is preferably cDNA of recombinant viral RNA encoding a sequence in which the nucleic acid of the present invention is inserted into the untranslated region ligated downstream of the coding region of the P gene in the BDV genome or a sequence in which either the G gene or M gene, or both are disrupted in the BDV genome.

In particular, (a) the cDNA of recombinant viral RNA is more preferably cDNA of recombinant viral RNA having a sequence in which either the G gene or M gene, or both are disrupted in the BDV genome and having a sequence in which the nucleic acid of the present invention is inserted into the untranslated region ligated downstream of the coding region of the P gene. (a) The cDNA of recombinant viral RNA is further preferably cDNA of recombinant viral RNA having a sequence in which the G gene is disrupted in the BDV genome and having a sequence in which the nucleic acid of the present invention is inserted into the untranslated region ligated downstream of the coding region of the P gene. By disrupting the G gene, the recombinant virus produced from the virus vector does not spread to cells other than the cells into which it has been introduced, making the recombinant virus less pathogenic and highly safe.

As (a) the cDNA of recombinant viral RNA, it is also possible to use cDNA encoding a recombinant BDV genome in which the nucleic acid of the present invention is inserted in the untranslated region between the coding region of the P gene and the coding region of the M gene.

The BDV genome may be any genome of genes of a virus belonging to the family Bornaviridae or a variant thereof. Examples of viruses belonging to the family Bornaviridae include He80, H1766, Strain V, huP2br, and the like. Examples of variants thereof include No/98, Bo/04w, HOT6, and the like.

(a) The cDNA of recombinant viral RNA may be cDNA of a recombinant BDV genome having a sequence in which the G gene in the BDV genome is disrupted and the G gene in avian bornavirus genome genes is inserted (see JP2018-148865A). This allows for higher production capacity and recovery of the resulting recombinant virus and efficient introduction of the nucleic acid of the present invention.

The ribozyme in (b) the DNA encoding a ribozyme may be any ribozyme having a sequence capable of cleaving the nucleic acid of the present invention transcribed from (a) the cDNA of recombinant viral RNA. Examples of (b) the DNA encoding a ribozyme include DNA, such as cDNA encoding a ribozyme, such as hammerhead ribozyme (HamRz), hepatitis delta virus ribozyme (HDVRz), hairpin ribozyme, or artificial ribozyme. Of these ribozymes, hammerhead ribozyme (HamRz) and hepatitis delta virus ribozyme (HDVRz) are preferable. It is particularly preferred that (b1) DNA (preferably cDNA) encoding HamRz is located upstream of (a) the cDNA of recombinant BDV genome, i.e., on the 3' end side, and that (b2) a DNA (preferably cDNA) sequence encoding HDVRz is located downstream of (a), i.e., on the 5' end side. This improves the efficiency of expressing the nucleic acid of the present invention in cells.

Examples of (c) the promoter sequence include an RNA polymerase II promoter sequence, an RNA polymerase I promoter sequence, and a T7 polymerase promoter sequence. Of these, an RNA polymerase II promoter sequence is preferable. Examples of RNA polymerase II promoters include a CMV promoter and a CAGGS promoter. Of these, a CAGGS promoter is particularly preferable.

The bornavirus vector of the present invention may further contain the whole or part of the SV40 viral replication origin/promoter region sequence, and one or more of the following: nucleic acid sequences encoding an enhancer, an activator (e.g., a trans-acting factor), a chaperon, and a processing protease. The virus vector of the present invention may be linear DNA or circular DNA. When the virus vector of the present invention is introduced into cells, the virus vector is preferably in a circular form.

The recombinant virus of the present invention is characterized by comprising RNA encoded by the bornavirus vector described above. The recombinant virus of the present invention preferably comprises RNA encoded by the bornavirus vector described above, the N protein of BDV, the P protein of BDV, and the L protein of BDV. Since the recombinant virus of the present invention comprises the recombinant viral RNA described above, the recombinant virus of the present invention is a persistently infecting recombinant virus that can persistently express the antibody or antibody fragment of the present invention after infection of cells. The recombinant virus may contain, if necessary, the G protein of BDV or a coat protein of another virus, and may further contain the M protein of BDV.

The recombinant virus of the present invention can be prepared by, for example, a method comprising the steps of introducing into a cell in vitro the bornavirus vector described above together with a plasmid or plasmids expressing the N gene, P gene, and L gene of BDV as a helper plasmid, and culturing the cells in which the bornavirus vector and the helper plasmid have been introduced, to produce a recombinant virus. In this case, when a G gene-deficient and M gene-deficient bornavirus vector is used, it is preferred that a plasmid expressing the coat gene of the virus and a plasmid expressing the M gene of BDV are introduced into a cell in vitro together with the plasmid or plasmids expressing the N gene, P gene, and L gene of BDV.

The cell of the present invention is a cell into which the nucleic acid of the present invention has been introduced by infection with the recombinant virus described above. By infecting a cell with the recombinant virus of the present invention, the nucleic acid of the present invention incorporated into the virus can be introduced into the cell.

The cell of the present invention is not particularly limited. Examples include oligodendrocyte progenitor cells, neural stem cells, mesenchymal stem cells (e.g., mesenchymal stem cells derived from bone marrow, dental pulp, and adipose), and the like. These can be suitably used for the treatment and prevention of amyotrophic lateral sclerosis. In addition to oligodendrocyte progenitor cells, neural stem cells, and mesenchymal stem cells, pluripotent stem cells, such as iPS cells (induced pluripotent stem cells), ES cells (embryonic stem cells), and Muse cells, glial neural progenitor cells, dopamine neural progenitor cells, microglia, and neural progenitor cells can be used as cells to be infected with the recombinant virus. Bornavirus vectors have high efficiency of introduction into stem cells and allow induction of differentiation while maintaining gene expression. Thus, cells obtained by infecting pluripotent stem cells, such as iPS cells, ES cells, and Muse cells, with the recombinant virus of the present invention and then inducing them to differentiate into oligodendrocyte progenitor cells, neural stem cells, mesenchymal stem cells, etc. also maintain expression of the antibody or antibody fragment described above. Therefore, the cell of the present invention also includes cells obtained by inducing differentiation from pluripotent stem cells infected with the recombinant virus (e.g., oligodendrocyte progenitor cells, neural stem cells, neural progenitor cells, and mesenchymal stem cells). Examples of the cell include cells of mammals, such as humans, monkeys, mice, rats, dogs, cats, and rabbits, with human cells being preferable. The cell may be autologous or allogeneic.

The method for infecting a cell with the recombinant virus in vitro preferably comprises preparing a diluted solution obtained by serially diluting the recombinant virus with, for example, a culture medium such as Dulbecco's Modified Eagle Medium or a phosphate buffered saline. The concentration of the recombinant virus in the diluted solution is appropriately selected according to the type of cell to be infected with the virus etc. Cells are infected with the recombinant virus by using the diluted solution.

The pharmaceutical composition of the present invention is administered to mammals, including humans, is characterized by comprising the cell described above, and is effective in treating and/or preventing diseases in which mutant SOD1 accumulates, in particular, amyotrophic lateral sclerosis.

The pharmaceutical composition of the present invention may optionally contain a biologically acceptable carrier, an excipient, and the like, depending on the use form. The pharmaceutical composition of the present invention can be produced by a conventional method. For example, the pharmaceutical composition of the present invention can be used nasally as a spray or the like, or parenterally in the form of an injection, such as a sterile solution or suspension, with water or other pharmaceutically acceptable liquids. The pharmaceutical composition of the present invention can also be used in combination with other known low-molecular-weight drugs. Examples of such drugs for ALS include riluzole, edaravone, and the like.

The amount of the cell, which is an active ingredient in the pharmaceutical composition of the present invention, is appropriately selected depending on the dosage form, route of administration, etc., and is generally about 1.0×10³ to 1.0×10¹⁰ cells, and preferably about 1.0×10⁴ to 1.0×10⁹ cells, in the total amount of the preparation.

The pharmaceutical composition of the present invention may be administered systemically or locally. The administration method is not particularly limited. The route of administration includes, for example, intrathecal, intraparenchymal, or intravenous injection or drip infusion, intranasal administration, and the like. Oligodendrocyte progenitor cells and neural stem cells can be administered intrathecally or intraparenchymally, and mesenchymal stem cells can be administered intrathecally, intraparenchymally, intravenously, or intranasally. The dose of the pharmaceutical composition of the present invention can be appropriately determined finally according to a doctor's judgement in consideration of the type of dosage form, the administration method, the age and body weight of the patient, the symptoms of the patient, and the like.

The pharmaceutical composition of the present invention can be used for the treatment of diseases in which mutant SOD1 accumulates because the administered cells secrete the antibody or antibody fragment and degrade and remove misfolded SOD1. The diseases in which mutant SOD1 accumulates are not particularly limited. Examples include amyotrophic lateral sclerosis and the like.

Cells expressing an antibody or antibody fragment capable of binding to a mutant SOD1 protein can be created by preparing a recombinant virus comprising RNA encoded by the bornavirus vector of the present invention by using the bornavirus vector, and further infecting cells with the recombinant virus. By administering the pharmaceutical composition of the present invention to transplant the cells, ALS having mutations in the SOD1 gene can be treated and prevented.

### Examples

Examples are provided below to describe the present invention in more detail. However, the present invention is not limited to these Examples.

### Experimental Method

### Anti-SOD1 antibody

The amino acid sequences and base sequences of VH and VL of the anti-SOD1 antibody (scFv) capable of binding to a mutant SOD1 protein used in the following experiments are shown below. The underlined parts are sequentially CDR1, CDR2, and CDR3.
VH (amino acid sequence) 
QVTLKESGPGILQPSQTLSLTCSFSGFSLNTSGMGVGWIRQPSGRVLEWLAHIWWDDDKRYNPAL
KSRLTISKDTSTNQVFLKIASVDTTDTATYYCARLGYAMDYWGHGTSVTVS (SEQ ID NO:
8)
VL (amino acid sequence) 
DIVMTQSQKFMSTSLGDRVSVTCKASQNVGTNVAWYQQKPGQSPKALIYSASYRYSGVPDRFTGS
GSGTDFTLTISNVQSEDLAEYFCQQYYIYPYTFGGGTKLEI (SEQ ID NO: 9)
VH (base sequence) (Test Examples 1 to 3) 
CAGGTTACTCTGAAAGAGTCTGGCCCTGGGATATTGCAGCCCTCCCAGACCCTCAGTCTGACTTG
TTCTTTCTCTGGGTTTTCACTGAACACTTCTGGTATGGGTGTAGGCTGGATTCGTCAGCCTTCAG
GGAGGGTTCTGGAGTGGCTGGCACACATTTGGTGGGATGATGACAAGCGCTATAACCCAGCCCTG
AAGAGCCGACTGACAATCTCCAAGGATACCTCCACCAACCAGGTATTCCTCAAGATCGCCAGTGT
GGACACTACAGATACTGCCACATACTACTGTGCTCGACTTGGCTATGCTATGGACTACTGGGGTC
ACGGAACCTCAGTCACCGTCTCC (SEQ ID NO: 10)
VL (base sequence) (Test Examples 1 to 3) 
GACATTGTGATGACCCAGTCTCAAAAATTCATGTCCACATCACTAGGAGACAGGGTCAGCGTCAC
CTGCAAGGCCAGTCAGAATGTGGGTACTAATGTAGCCTGGTATCAACAGAAACCAGGGCAATCTC
 
CTAAAGCACTGATTTACTCGGCATCCTACCGGTACAGTGGAGTCCCTGATCGCTTCACAGGCAGT
GGATCTGGGACAGATTTCACTCTCACCATCAGCAATGTGCAGTCTGAAGACTTGGCAGAGTATTT
CTGTCAGCAATATTACATCTATCCGTACACGTTCGGAGGGGGGACCAAGCTGGAAATA (SEQ
ID NO: 11)
VH (base sequence) (Test Examples 4 to 6) 
CAGGTTACTCTGAAGGAGTCTGGCCCTGGGATACTGCAGCCCTCACAGACCCTGAGTCTGACTTG
TTCCTTCTCTGGATTTTCACTGAACACCTCTGGTATGGGTGTAGGCTGGATTCGTCAGCCTTCCG
GGAGGGTTCTGGAGTGGCTGGCTCACATCTGGTGGGATGATGATAAGCGCTATAACCCTGCCCTG
AAGAGCCGACTGACCATCTCTAAGGATACCTCCACCAACCAGGTATTCCTCAAGATCGCCAGTGT
GGACACCACCGATACTGCTACATACTACTGTGCTCGTCTTGGCTATGCTATGGACTACTGGGGTC
ACGGAACCTCAGTCACCGTCTCC (SEQ ID NO: 12)
VL (base sequence) (Test Examples 4 to 6)

GACATTGTGATGACCCAGTCTCAAAAATTCATGTCCACCTCACTTGGAGACCGAGTCTCAGTCAC
CTGCAAGGCTTCCCAGAATGTGGGTACAAATGTAGCCTGGTATCAGCAGAAACCAGGACAGTCTC
CTAAGGCACTGATTTACTCGGCTTCCTACCGTTACTCCGGAGTCCCTGATCGTTTCACAGGTAGT
GGATCTGGAACAGATTTCACCCTGACCATCAGCAATGTTCAGTCTGAAGATTTGGCAGAGTATTT
CTGCCAGCAATATTACATCTATCCGTACACGTTCGGAGGAGGAACCAAGCTGGAAATT (SEQ
ID NO: 13)

### Production of pCAG-Fct p/mΔG LL GFP-scFv plasmid into which anti-SOD1 antibody gene is inserted

A pCAG-Fct p/m tandem GFP ΔG LL plasmid was produced by the same procedure as in JP2018-148865A. The plasmid carries two cassettes, i.e., a cassette of AscI and AsiSI sites and a cassette of BstBI and PacI sites, between the P gene and M gene, as foreign gene insertion cassettes. PCR (98°C for 10 seconds, 55°C for 5 seconds, 72°C for 5 seconds; 30 cycles) was performed using an eGFP gene expression plasmid (pcDNA3-eGFP) as a template, primers, and PrimeSTAR (trademark) MAX DNA polymerase (Takara Bio Inc.). PCR products were recombined with the pCAG-Fct p/m tandem GFP ΔG LL using AscI and AsiSI. Subsequently, PCR (98°C for 10 seconds, 55°C for 5 seconds, 72°C for 5 seconds; 30 cycles) was performed using anti-SOD1 antibody scFv cDNA as a template, primers, and PrimeSTAR MAX DNA polymerase (Takara Bio Inc.). PCR products were recombined with the pCAG-Fct p/m tandem GFP ΔG LL using BstBI and PacI, thereby obtaining pCAG-Fct p/mΔG LL GFP-scFv (Fig. 1). Myc is bound to the scFv here.

### Production of recombinant virus

The pCAG-Fct p/mΔG LL GFP-scFv and helper plasmids (N gene expression plasmid, P gene expression plasmid, L gene expression plasmid, and G gene expression plasmid: pcN, pCXN2-P, pcL, and pcG, respectively) were introduced into 293T cells by using TransIT-293 Transfection Reagent (Mirus Bio), Lipofectamine (trademark) 2000 (Invitrogen), and FuGENE (trademark) 6 Transfection Reagent (Promega K.K.). The amounts of the virus vector and the like used for introduction were as follows: relative to 1×10⁴ to 1×10⁶ 293T cells, 1 to 4 µg of the pCAG-Fct p/mΔG LL GFP-scFv was used, and the helper plasmids were added in the following proportions: pcN (0.125 to 0.5 µg), pCXN2-P (0.125 to 0.05 µg), pcL (0.125 to 0.5 µg), and pcG (0.05 to 0.125 µg). In this case, helper plasmids into which gene sequences of not only borna disease virus 1 (BoDV-1), but also borna disease virus 2 (BoDV-2) have been inserted may be used for pcN and pCXN2-P.

After the introduction of the virus vector and the helper plasmids into 293T cells, the cells were cultured at 37°C. Three days after introduction, the cells were passaged and further co-cultured with Vero cells constitutively expressing the G gene (Vero-G cells). The Vero-G cells were collected as recombinant virus-infected Vero cells.

### Recovery of recombinant virus (bornavirus vector)

The recombinant virus-infected Vero-G cells prepared above were washed twice with PBS buffer and treated with 2.5 mg/l-trypsin/1 mmol/l-EDTA solution (Nacalai Tesque, Inc.) to suspend the cells, followed by centrifugation at 3,500 rpm for 5 minutes to collect the infected cells. After centrifugation, the infected cells were suspended in 500 µL of Opti-MEM (Thermo Fisher Scientific Inc.) and sonicated using BIORUPTOR II (Sonic Bio Co., Ltd.). After sonication, the disrupted cell liquid was centrifuged at 1,200 × g for 25 minutes at 4°C, and the supernatant was collected as a virus vector solution and stored at -80°C.

### Collection and culture of oligodendrocyte progenitor cells (OPCs) from rats

Newborn (days 0-2) wild-type SD rats were euthanized under isoflurane inhalation anesthesia, and a primary culture system was prepared. Mixed glia (a mixture of astrocytes, microglia, and OPCs) isolated from the cerebral cortex of two rats per flask were cultured in 75-cm EasyFlasks (Nunc) at 37°C for 2 weeks and shaken for 24 hours to isolate OPCs. The isolated OPCs were seeded in OPC proliferation medium (a mixture of 49 mL of Neurobasal medium (Invitrogen), 1 mL of serum-free B27 Supplement (Invitrogen), 5 µL of Human PDGF-AA (Peprotech), and 5 µL of FGF-Basic (Peprotech)) at a concentration of two billion cells per 1 ml and cultured at 37°C.

### Introduction of vector into OPCs

After removing the culture supernatant of the OPCs seeded and cultured in a 10-cm dish, the virus vector solution prepared above was added to the OPCs, and the mixture was allowed to stand at 37°C for 1 hour. After that, washing was performed using a sufficient amount of Opti-MEM, and 2.5 mL of OPC proliferation medium (a mixture of 49 mL of Neurobasal medium (Invitrogen), 1 mL of serum- free B27 Supplement (Invitrogen), 5 µL of Human PDGF-AA (Peprotech), and 5 µL of FGF-Basic (Peprotech)) was added, followed by culturing at 37°C.

### Transplantation of bornavirus vector-transduced OPCs

The infected OPCs after culturing in the 10-cm dish were detached with Accumax (Nacalai Tesque, Inc.) and centrifuged at 500 G for 10 minutes, and adjustment was made so that 1.0×10⁶ OPCs were dissolved in 10 µl of OPC proliferation medium. The occipital region of a 19-week-old SOD1H46R rat was incised under isoflurane inhalation anesthesia, and 10 µl of the above OPC dissolved medium was administered between the occipital bone and the first cervical vertebra.

### Test Example 1

The virus vector collected by the above method was inoculated into OPCs. Fig. 2 is a fluorescence microscopic image of the OPCs 3 days after inoculation. The GFP inserted into the virus vector was expressed in the OPCs, and GFP fluorescence was observed.

The culture supernatant of the OPCs was collected and centrifuged at 3,500 rpm at 4°C for 5 minutes. To the culture supernatant, an equal amount of 2×SDS-sample buffer (125 mM Tris-HCl buffer (Tris, Nacalai Tesque, Inc.) (HCl, FUJIFILM Wako Pure Chemical Corporation) containing 10% mercaptoethanol (FUJIFILM Wako Pure Chemical Corporation), 4% SDS (FUJIFILM Wako Pure Chemical Corporation), 0.3 M sucrose (FUJIFILM Wako Pure Chemical Corporation), and 0.01% BPB (FUJIFILM Wako Pure Chemical Corporation)) was added, followed by heating at 95°C for 5 minutes. After heating, SDS-PAGE was performed using the prepared sample and Any kD Mini-PROTEAN TGX precast gel (Bio-Rad Laboratories). After electrophoresis, transfer was performed onto a PVDF membrane using Trans-Blot Turbo PVDF Transfer Pack (Bio-Rad Laboratories) by semi-dry blotting. After protein transfer onto the membrane, blocking was performed using Blocking One (Nacalai Tesque, Inc.), followed by washing with TBS-T buffer containing 0.1% Tween(trademark)-20 (FUJIFILM Wako Pure Chemical Corporation).

To detect the scFv, the PVDF membrane after the transfer was reacted with anti-Myc monoclonal antibody 9E10 (Merck Millipore) diluted with CanGet Signal Immunoreaction Enhancer Solution 1 (Toyobo Co., Ltd.) and allowed to stand at room temperature for 1 hour. Thereafter, washing was performed three times with TBS-T, and the PVDF membrane was reacted with a peroxidase-conjugated anti-mouse IgG antibody (Jackson ImmunoResearch) diluted with CanGet Signal Immunoreaction Enhancer Solution 2 (Toyobo Co., Ltd.) and allowed to stand at room temperature for 1 hour. Thereafter, washing was performed with TBS-T, and the scFv in the OPC supernatant was detected using Amersham ECL Prime (GE Healthcare) and ImageQuant LAS 4000 mini (GE Healthcare).

Fig. 3 shows the results. A band (scFv) was detected in the culture supernatant of the OPCs into which the vector encoding the scFv had been introduced (lane 3), and the intensity of the band detected in culture with the addition of Brefeldin A, i.e., an extracellular secretion inhibitor (lane 4), was weaker than that without the addition of Brefeldin A. These results suggested that the scFv derived from the bornavirus vector was secreted in the culture supernatant of the OPCs via an intracellular transport mechanism.

### Test Example 2

Culture medium (NI; n=10), OPCs into which a bornavirus vector carrying GFP had been introduced (OPC BoDV GFP; n=10), and OPCs into which a bornavirus vector carrying GFP and scFv had been introduced (OPC BoDV scFv; n=10) were administered intrathecally to H46R mutant SOD1 transgenic rats. The number of OPCs administered was 1.0×10⁶ cells per individual. The OPCs were suspended in 10 µL of medium and administered. Each group of transgenic mice was subjected to a survival rate test, measurement of body weight change, an inclined plate test, a grip strength test, and a hindlimb reflex test.
Survival rate test: The survival rate of each group was shown with a Kaplan-Meier curve.
Body weight change: The body weight of each individual was measured on each measurement day, and weight reduction was plotted with the body weight (g) on the first day of measurement taken as 100%.
Inclined plate test: Each individual was placed on a flat plate, and the maximum angle at which the individual could maintain the posture without falling for 5 seconds or more was measured and used as an index of muscle strength.
Grip strength test: The forelimbs of each individual were placed on a grip strength meter for rats and mice (Muromachi Kikai Co., Ltd.) while the individual was held by the tail, and the tail was slowly pulled. This was repeated five times, and the maximum value was taken as the measured value for that day.
Hindlimb reflex test: Each individual was lifted by the tail, and the progression of ALS symptoms was analyzed using the reflex response of limb extension. As an index of paralysis, scoring was performed on each measurement day as follows: no paralysis was scored as 0, extension of one of the four limbs was scored as 1, extension of all of the four limbs was scored as 4, and so on.

Fig. 4 shows the results. In the OPC scFv group, the survival rate was improved (Fig. 4A), and the body weight change was also improved (Fig. 4B), compared with the NI group and the OPC GFP group. The results of the inclined plate test (Fig. 4C), grip strength test (Fig. 4D), and hindlimb reflex test (Fig. 4E) as indexes of ALS symptoms suggested that ALS symptoms were alleviated in the OPC scFv group in all of the tests.

### Test Example 3

Eight weeks after OPC transplantation, tissue was collected from each individual, and relative quantitation of the amount of SOD1 in the lumbar spinal cord was performed by western blotting. A fraction obtained by lysing the collected tissue with RIPA buffer (containing 50 mM of Tris-HCL (pH of 7.7), 150 mM of NaCl, 1% Nonidet P40 substitute, 0.5% sodium deoxycholate, and 0.1% SDS) and performing extraction was designated as "soluble fraction," a fraction obtained by subjecting the residue after lysis with the RIPA buffer to extraction with 2% SDS was designated as "insoluble fraction," and western blotting was performed using anti-Myc monoclonal antibody 9E10 (Merck Millipore) in the same manner as above. Aggregated SOD1, which causes ALS, is extracted in the insoluble fraction. As an endogenous control for the same sample, beta-actin was also detected using an anti-beta-actin antibody (Sigma-Aldrich).

Figs. 5 and 6 show the results. The SOD1/beta-actin ratio was calculated based on the intensity of each detected band, and the relative SOD1 amount is shown in a graph. The SOD1 amount in each OPC administration group is shown on the assumption that the SOD1/beta-actin in the NI group is 1.0. The results showed that detected SOD1 was significantly reduced in the OPC scFv group compared with the NI group and the OPC GFP group, suggesting that SOD1 was degraded in vivo by the scFv.

### Production of pCAG-FctHe80 p/lΔMG scFv plasmid into which anti-SOD1 antibody gene is inserted

A pCAG-FctHe80 p/lΔMG scFv plasmid was produced by the same procedure as in Fujino K et al. (Microbiol Immunol. 2017 Sep; 61(9):380-386.doi: 10.1111/1348-0421.12505.). The plasmid carries a cassette of BstBI and PacI sites between the P and L genes as a foreign gene insertion cassette. PCR (98°C for 10 seconds, 55°C for 5 seconds, 72°C for 5 seconds; 35 cycles) was performed using anti-SOD1 antibody scFv cDNA as a template, primers, and PrimeSTAR MAX DNA polymerase (Takara Bio Inc.). PCR products were recombined with a pCAG-FctHe80 p/lΔMG plasmid using BstBI and PacI, thereby obtaining a recombinant pCAG-FctHe80 p/lΔMG scFv plasmid (Fig. 7). Myc is bound to the scFv here.

### Production of M gene-deficient and G gene-deficient recombinant virus

The pCAG-FctHe80 p/lΔMG scFv and helper plasmids (N gene expression plasmid, P gene expression plasmid, L gene expression plasmid, M gene expression plasmid, and G gene expression plasmid: pcN, pCXN2-P, pcL, pcM, and pcG, respectively) were introduced into 293T cells by using TransIT-293 Transfection Reagent (Mirus Bio). The amounts of the virus vector and the like used for introduction were as follows: relative to 1×10⁴ to 1×10⁶ 293T cells, 1 to 4 µg of the pCAG-FctHe80 p/lΔMG scFv was used, and the helper plasmids were added in the following proportions: pcN (0.125 to 0.5 µg), pCXN2-P (0.125 to 0.05 µg), pcL (0.125 to 0.5 µg), pcM (0.05 to 0.125 µg), and pcG (0.05 to 0.125 µg). In this case, helper plasmids into which gene sequences of not only borna disease virus 1 (BoDV-1), but also borna disease virus 2 (BoDV-2) have been inserted may be used for pcN and pCXN2-P.

After the introduction of the virus vector and the helper plasmids into 293T cells, the cells were cultured at 37°C. Three days after introduction, the cells were passaged and further co-cultured with Vero cells constitutively expressing the M and G genes (Vero-MG cells). The Vero-MG cells were collected as recombinant virus-infected Vero cells.

### Recovery of M gene-deficient and G gene-deficient recombinant virus

The recombinant virus-infected Vero-MG cells prepared above were washed twice with 20 mM HEPES buffer (Thermo Fisher Scientific Inc., pH of 7.5), and 20 mM HEPES (pH of 7.5)/250 mM MgCl₂/1% FCS buffer was added, followed by allowing the mixture to stand at 37°C for 90 minutes. Thereafter, the added buffer was collected, and the supernatant obtained by centrifugation at 3,500 rpm at 4°C for 5 minutes was stored at -80°C as a virus vector solution.

### Introduction of bornavirus vector into mesenchymal stem cells

Human bone marrow-derived mesenchymal stem cells (MSCs) (PromoCell, #C-12974) were seeded in 6-well plates coated with RetroNectin (trademark) (Takara Bio Inc.). After removing the culture supernatant of the mesenchymal stem cells cultured in Mesenchymal Stem Cell Growth Medium XF (PromoCell), the virus vector solution prepared above was added to the mesenchymal stem cells at an MOI of 0.25 or 0.5, and the plates were allowed to stand at 37°C for 90 minutes. Thereafter, washing was performed twice with a sufficient amount of Mesenchymal Stem Cell Growth Medium XF, and culturing was continued at 37°C.

### Test Example 4

The culture media were collected 3 days and 6 days after inoculation, and each supernatant obtained by centrifugation at 3,500 rpm at 4°C for 5 minutes was concentrated 10-fold using Amicon Ultra 10K (Merck Millipore). To the concentrated culture supernatant, an equal amount of 2×SDS-sample buffer (125 mM Tris-HCl buffer (Tris, Nacalai Tesque, Inc.) (HCl, FUJIFILM Wako Pure Chemical Corporation) containing 10% mercaptoethanol (FUJIFILM Wako Pure Chemical Corporation), 4% SDS (FUJIFILM Wako Pure Chemical Corporation), 0.3 M sucrose (FUJIFILM Wako Pure Chemical Corporation), and 0.01% BPB (FUJIFILM Wako Pure Chemical Corporation)) was added, followed by heating at 95°C for 5 minutes. After heating, SDS-PAGE was performed using the prepared sample and Any kD Mini-PROTEAN TGX precast gel (Bio-Rad Laboratories). After electrophoresis, transfer was performed onto a PVDF membrane using Trans-Blot Turbo PVDF Transfer Pack (Bio-Rad Laboratories) by semi-dry blotting. After protein transfer onto the membrane, blocking was performed using Blocking One (Nacalai Tesque, Inc.) followed by washing with TBS-T buffer containing 0.1% Tween-20 (FUJIFILM Wako Pure Chemical Corporation).

To detect the scFv, the PVDF membrane after the transfer was reacted with anti-Myc monoclonal antibody 9E10 (Merck Millipore) or anti-α-tubulin antibody (Sigma-Aldrich) diluted with CanGet Signal Immunoreaction Enhancer Solution 1 (Toyobo Co., Ltd.) and allowed to stand at room temperature for 1 hour. Thereafter, washing was performed three times with TBS-T, and the PVDF membrane was reacted with a peroxidase-conjugated anti-mouse IgG antibody (Jackson ImmunoResearch) diluted with CanGet Signal Immunoreaction Enhancer Solution 2 (Toyobo Co., Ltd.) and allowed to stand at room temperature for 1 hour. Thereafter, washing was performed with TBS-T, and the scFv secreted in the mesenchymal stem cell supernatant was detected using Amersham ECL Prime (GE Healthcare) and ImageQuant LAS 4000 mini (GE Healthcare).

Fig. 8 shows the results. Bands (scFv) were detected in the culture supernatants of the mesenchymal stem cells into which the vector encoding the scFv had been introduced (lanes 3 to 6), and the intensity of the band at a MOI of 0.5 detected was increased compared with that at a MOI of 0.25, in a manner dependent on the amount of the vector introduced. The non-transduced MSCs in lane 1 indicate MSCs into which no bornavirus vector was introduced, and the transduced MSCs (Ctrl) in lane 2 indicate MSCs into which a bornavirus vector encoding GFP was introduced in place of the vector encoding the scFv. The results suggested that the scFv derived from the bornavirus vector was secreted in the culture supernatants of the mesenchymal stem cells.

### Production of lentivirus vector into which anti-SOD1 antibody gene is inserted

CSII-CMV scFv and CSII-EF scFv plasmids were produced by the same procedure as in Miyoshi, H et al. (Gene delivery to hematopoietic stem cells using lentiviral vectors. Methods Mol. Biol. 246:429-38, 2004. PMID: 14970608). PCR (98°C for 10 seconds, 55°C for 5 seconds, 72°C for 5 seconds; 35 cycles) was performed using anti-SOD1 antibody scFv cDNA as a template, primers, and PrimeSTAR MAX DNA polymerase (Takara Bio Inc.). PCR products were recombined with a CSII-CMV MSC or CSII-EF MCS plasmid using XhoI and BamHI, thereby obtaining CSII-CMV scFv and CSII-EF scFv plasmids.

The lentivirus vector CSII-CMV scFv or CSII-EF scFv plasmid, a pCAG-HIVgp plasmid, a pCMV-VSVG plasmid, and a pRSV-rev plasmid were introduced into 293T cells by using TransIT-293 Transfection Reagent (Mirus Bio). The amounts of the virus vector and the like used for introduction were as follows: into 1×10⁵ to 1×10⁷ 293T cells, 1 to 4 µg of the lentivirus vector CSII-CMV scFv or CSII-EF scFv, 0.5 to 2 µg of pCAG-HIVgp, 0.5 to 2 µg of pCMV-VSVG, and 0.5 to 2 µg of pRSV-rev were individually introduced. After introduction of the plasmids into 293T cells, the cells were cultured at 37°C. Three days after introduction, the culture supernatant was collected as a lentivirus vector solution. To the collected lentivirus vector solution, Lenti-X Concentrator (Clontech) was added in a 1/3 amount, followed by suspension. Subsequently, the mixture was allowed to stand at 4°C for 16 hours. Thereafter, the mixture was centrifuged at 1,500 × g for 45 minutes to remove the supernatant, and the pellet was resuspended in PBS to concentrate the lentivirus vector solution 10-fold to 100-fold. The concentrated lentivirus vector solution was stored at -80°C.

### Test Example 5

The pCAG-FctHe80 p/lΔMG scFv and the CSII-CMV scFv and CSII-EF scFv lentivirus vectors were individually introduced into human bone marrow-derived mesenchymal stem cells cultured in Mesenchymal Stem Cell Growth Medium XF, at an MOI of 0.2. RNA was extracted from the cells using an RNeasy Mini Kit (QIAGEN) 2 and 4 days after introduction. After cDNA was synthesized using the extracted RNA and a Verso cDNA Synthesis Kit (Thermo Fisher Scientific Inc.), RT-qPCR was performed using scFv or GAPDH gene-specific primers and probe, and Luna Universal qPCR Master Mix (New England Biolabs) with a CFX96 Touch Real-Time PCR Detection System (Bio-Rad Laboratories).

A relative comparison of the amount of scFv mRNA of each sample was performed based on the amount of scFv mRNA relative to GAPDH as an endogenous control 2 days after introduction of the bornavirus vector. Fig. 9 shows the results. Two days after introduction, the mRNA expression level in the case of using the bornavirus vector was lower than that in the cases of using the lentivirus vectors; however, 4 days after introduction, the mRNA expression level in the case of using the bornavirus vector was 1.5 times that in the cases of using the lentivirus vectors.

pCAG-FctHe80 p/lΔMG scFv-HiBit and CSII-CMV scFv-HiBit and CSII-EF scFv-HiBit lentivirus vectors, all of which encode a gene with a HiBit tag added to the C-terminus of the scFv, were produced by the same procedure as above. Each vector was individually introduced at an MOI of 0.2 into human bone marrow-derived mesenchymal stem cells seeded in 6-well plates, and 100 µL of culture supernatant was collected every 2 days from day 0 to day 24 after introduction. The secreted protein was quantified over time by chemiluminescence using each collected culture supernatant and the NanoGlo HiBit Extracellular Detection System (Promega) with a GloMAX Discover Microplate Reader (Promega). Fig. 10 shows the results. Four days after introduction, chemiluminescence that was almost equivalent to that of the lentivirus vector-transduced cells was detected, suggesting that the amount of scFv secreted was similar. Furthermore, no significant decrease in luminescence was observed for 24 days after introduction, suggesting that the scFv was continuously secreted from the mesenchymal stem cells.

### Test Example 6

By the same procedure as above, each virus vector was individually introduced into human bone marrow-derived mesenchymal stem cells at an MOI of 0.2, and DNA was recovered from the cells 2 days and 4 days after introduction. RNA was degraded by adding RNase A (QIAGEN) when cellular DNA was extracted using a Qiamp DNA Mini Kit. After DNA purification, the DNA concentration was measured using Qubit dsDNA HS Assay (Thermo Fisher Scientific Inc.). scFv DNA was quantified by qPCR using 200 ng of DNA and a primer pair and probe specific to the scFv gene. Fig. 11 shows the results. scFv DNA was detected from the lentivirus vector-transduced cells, whereas the scFv DNA amount in similarly purified DNA of the bornavirus vector-transduced cells was below the detection limit. The results suggested that in the bornavirus vector-transduced mesenchymal stem cells, the amount of DNA of the vector-derived foreign gene created after introduction was significantly lower than that in the cases of using the lentivirus vectors, and that the probability of integration into the host genome was extremely low.

## Claims

1. A bornavirus vector comprising a nucleic acid encoding an antibody or antibody fragment capable of binding to a mutant SOD1 protein,
the antibody or antibody fragment comprising:
a heavy-chain variable region comprising a heavy-chain CDR1 consisting of an amino acid sequence GFSLNTSGMG (SEQ ID NO: 1), a heavy-chain CDR2 consisting of an amino acid sequence IWWDDDK (SEQ ID NO: 2), and a heavy-chain CDR3 consisting of an amino acid sequence ARLGYAMDY (SEQ ID NO: 3), the heavy-chain variable region optionally having 3 or fewer amino acid substitutions; and/or
a light-chain variable region comprising a light-chain CDR1 consisting of an amino acid sequence QNVGGTN (SEQ ID NO: 4), a light-chain CDR2 consisting of an amino acid sequence SAS, and a light-chain CDR3 consisting of an amino acid sequence QQYYIYPYT (SEQ ID NO: 5), the light-chain variable region optionally having 3 or fewer amino acid substitutions.

2. The bornavirus vector according to claim 1, wherein the antibody fragment is a Fab, Fab', F(ab')₂, Fv, scFv, scFab, dsFv, ds-scFv, diabody, triabody, tetrabody, or minibody.

3. The bornavirus vector according to claim 1 or 2, wherein the antibody fragment is an scFv.

4. The bornavirus vector according to claim 1 or 2, wherein the antibody or antibody fragment is a humanized antibody or antibody fragment.

5. The bornavirus vector according to claim 1 or 2, comprising:
(a) cDNA of recombinant viral RNA having at least the N gene, X gene, P gene, and L gene of a Borna disease virus genome in the same order as in the Borna disease virus genome and having a sequence in which the nucleic acid is inserted into the untranslated region ligated downstream of the coding region of the P gene;
(b) DNA encoding a ribozyme; and
(c) a promoter sequence,
wherein (b) is located upstream and downstream of (a), and (a) and (b) are located downstream of (c).

6. A recombinant virus comprising RNA encoded by the bornavirus vector of claim 1 or 2.

7. A cell into which the nucleic acid has been introduced by infection with the recombinant virus of claim 6.

8. The cell according to claim 7, wherein the cell is an oligodendrocyte progenitor cell, glial neural progenitor cell, dopamine neural progenitor cell, microglia, neural progenitor cell, neural stem cell, mesenchymal stem cell, Muse cell, iPS cell, or ES cell.

9. A pharmaceutical composition comprising the cell of claim 7.

10. The pharmaceutical composition according to claim 9, which is for use in the treatment and/or prevention of amyotrophic lateral sclerosis.
